(19) Europäisches Patentamt
European Patent Office
Office européen des brevets

(11) Veröffentlichungsnummer: **0 195 196**
**B1**

# (12) EUROPÄISCHE PATENTSCHRIFT

(45) Veröffentlichungstag der Patentschrift: **06.09.89**

(51) Int. Cl.⁴: **C 07 C 118/00**

(21) Anmeldenummer: **86100977.7**

(22) Anmeldetag: **24.01.86**

(54) **Verfahren zur Herstellung von 2-Isocyanato-2,3-dehydrocarbon-säureestern.**

(30) Priorität: **11.03.85 DE 3508563**

(43) Veröffentlichungstag der Anmeldung:
**24.09.86 Patentblatt 86/39**

(45) Bekanntmachung des Hinweises auf die Patenterteilung:
**06.09.89 Patentblatt 89/36**

(84) Benannte Vertragsstaaten:
**AT BE CH FR GB IT LI LU NL SE**

(56) Entgegenhaltungen:
**DE-A-2 461 963**

**PATENT ABSTRACTS OF JAPAN, Band 6, Nr. 197 (C-128)[1075], 6. Oktober 1982; & JP - A - 57 108 055**
**CHEMICAL ABSTRACTS, Band 81, Nr. 18, 4. November 1974, Seite 585, Zusammenfassungsnr. 113967m, Columbus, Ohio, US; G. LA MONICA et al.: "Synthesis and reactions of isocyanate and carbamoyl complexes of rhenium"**
**CHEMICAL ABSTRACTS, Band 78, Nr. 21, 28. Mai 1973, Seite 374, Zusammenfassungsnr. 136165a, Columbus, Ohio, US; F.J. WEIGERT et al.: "Synthesis of aryl isocyanates from nitro compounds and carbon monoxide"**

(73) Patentinhaber: **Degussa Aktiengesellschaft, Weissfrauenstrasse 9, D-6000 Frankfurt am Main 1 (DE)**

(72) Erfinder: **Effenberger, Franz, Prof.Dr.Dipl.-Chem., Schatzweg 5, D-7000 Stuttgart (DE)**
Erfinder: **Kühlwein, Jürgen, Dipl.-Chem., Sebastian-Bach-Strasse 4, D-7050 Waiblingen 7 (DE)**
Erfinder: **Drauz, Karlheinz, Dr.Dipl.-Chem., Flurstrasse 5, D-6453 Freigericht 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von 2-Isocyanato-2,3-dehydrocarbonsäureestern (1-Alkoxycarbonyl-1-alkenyl-isocyanaten) der allgemeinen Formel

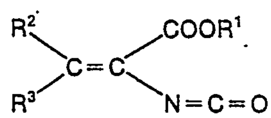

(I),

in der

$R^1$ einen geradkettigen oder verzweigten $(C_1–C_4)$-Alkylrest, einen Phenylrest oder einen Benzylrest,

$R^2$ Wasserstoff oder einen Methylrest und

$R^3$ Wasserstoff, einen geradkettigen oder verzweigten $(C_1–C_{16})$-Alkylrest, einen $(C_3–C_8)$-Cycloalkylrest, einen $(C_1–C_6)$-Alkylmercaptorest oder einen Phenylrest bedeuten.

Aus der europäischen Patentanmeldung 86 424 ist ein Verfahren zur Herstellung von 1-Alkenylisocyanaten bekannt, die keine weiteren funktionellen Gruppen enthalten. Dieses Verfahren, das einen Dehydrohalogenierungsschritt zur Ausbildung der C=C-Doppelbindung umfaßt, ist jedoch zur Herstellung der Verbindungen der allgemeinen Formel (I) nicht geeignet.

Das erfindungsgemäße Verfahren ist nun dadurch gekennzeichnet, daß man einen 2-Azidocarbonsäureester der allgemeinen Formel

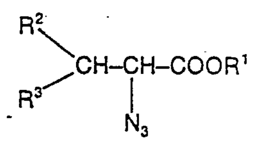

(II),

in der $R^1$, $R^2$ und $R^3$ die bereits angegebene Bedeutung haben, in einem inerten Lösungsmittel bei einer Temperatur zwischen 0 und 150 °C in Gegenwart eines Perrhenats mit Phosgen oder Diphosgen (Chlorameisensäuretrichlormethylester) umsetzt.

Die Verbindungen der allgemeinen Formel (I) sind bisher nicht beschrieben. Sie lassen sich in einer einfachen Reaktion in sehr hohen Ausbeuten mit Benzylalkohol oder mit tert.Butylalkohol in die entsprechenden Z- bzw. BOC-geschützten 2,3-Dehydro-2-amino-carbonsäureester umwandeln, die ihrerseits wertvolle Bausteine für die Synthese von Dehydropeptiden sind.

Durch das erfindungsgemäße Verfahren lassen sich die 2-Isocyanato-2,3-dehydrocarbonsäureester der allgemeinen Formel (I) in einem einstufigen Verfahren leicht und in annehmbaren Ausbeuten herstellen.

Die als Ausgangsmaterialien dienenden 2-Azido-carbonsäureester der allgemeinen Formel (II) können nach dem in Chem. Ber. 117, 1497–1512 (1984) beschriebenen Verfahren durch Umsetzung der entsprechenden 2-Chlor- oder 2-Bromcarbonsäureester mit einer wäßrigen Natriumazidlösung in Gegenwart eines Phasentransferkatalysators hergestellt werden. Als Beispiele seien genannt die Ester der
2-Azido-propionsäure, -buttersäure, -3-methylbuttersäure, -3-phenylpropionsäure, -valeriansäure, -4-methylpentansäure, -3-cyclopropylpropionsäure, -3-cyclopentylpropionsäure, -3-cyclohexylpropionsäure, -hexansäure, -heptansäure, -octansäure, -nonansäure, -decansäure, -undecansäure, -dodecansäure, -tridecansäure, -tetradecansäure, -pentadecansäure, -hexadecansäure, -heptadecansäure, -octadecansäure, -nonadecansäure oder -3-methylmercaptopropionsäure.

Für die Durchführung des erfindungsgemäßen Verfahren geeignete inerte Lösungsmittel sind beispielsweise Carbonsäurealkylester, wie Essigsäureethylester, Essigsäure-n-propylester, Essigsäureisopropylester, Essigsäure-n-butylester oder Propionsäureethylester; Ether, wie Dimethoxyethan, Dioxan oder Tetrahydrofuran; ferner Cyclohexan, Toluol oder Chlorbenzol.

Vorzugsweise wird das erfindungsgemäße Verfahren bei einer Temperatur zwischen 20 und 100 °C durchgeführt.

Als Katalysatoren geeignete Perrhenate sind insbesondere das Natrium- oder Kaliumperrhenat oder quartäre Ammoniumperrhenate, wie Tetraethylammoniumperrhenat, Tetrabutylammoniumperrhenat, Triethylbenzylammoniumperrhenat oder Tricaprylmethylammoniumperrhenat. Sie werden zweckmäßigerweise in einer Menge zwischen 0,005 und 10 Molprozent, vorzugsweise zwischen 0,1 und 3 Molprozent, bezogen auf die Menge an eingesetztem 2-Azido-carbonsäureester der allgemeinen Formel (II) angewandt.

Das Phosgen kann als solches oder als Diphosgen eingesetzt werden, da letzteres bei höherer Temperatur in Phosgen zerfällt:

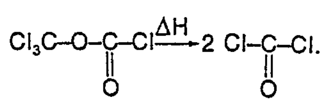


Das Phosgen wird zweckmäßigerweise in einer Menge von mindestens 1 Mol, das Diphosgen in einer Menge von mindestens 0,5 Mol, vorzugsweise zwischen 0,51 und 1,5 Mol, jeweils pro Mol eingesetztem 2-Azido-carbonsäureester der allgemeinen Formel (II) angewandt. Die zum Einsatz kommende molare Menge an Phosgen bzw. Diphosgen ist im übrigen nicht kritisch.

Bei Verwendung von Phosgen als solchem kann dieses entweder im Überschuß gasförmig durch das Reaktionsgemisch durchgeleitet oder in kondensierter Form zudosiert werden.

Unter Umständen kann es zweckmäßig sein, vor Beginn der Zugabe des Phosgens oder Diphosgens zum Reaktionsgemisch in das Lösungsmittel trockenen gasförmigen Chlorwasserstoff einzuleiten. Die Menge des Chlorwasserstoffs ist ebenfalls nicht kritisch. Sie kann bis zur Sättigungskonzentration im jeweiligen Lösungsmittel reichen.

Das erfindungsgemäße Verfahren kann beispielsweise so durchgeführt werden, daß man das

Perrhenat und Diphosgen im Lösungsmittel vorlegt, gegebenenfalls zusätzlich noch trockenen Chlorwasserstoff einleitet, und dann den 2-Azidocarbonsäureester der allgemeinen Formel (II), gelöst im gleichen Volumen des verwendeten Lösungsmittels, innerhalb von zehn Minuten bis einigen Stunden bei der gewählten Reaktionstemperatur zudosiert. Es wird in jedem Falle bis zur Beendigung der Stickstoffentwicklung bei der gewählten Reaktionstemperatur weitergerührt.

Es kann auch so verfahren werden, daß man den 2-Azido-carbonsäureester der allgemeinen Formel (II) zusammen mit dem Perrhenat im Lösungsmittel vorlegt, gegebenenfalls zusätzlich noch trockenen Chlorwasserstoff einleitet, und dann das kondensierte Phosgen oder das Diphosgen zudosiert oder gasförmiges Phosgen einleitet.

Nach beendeter Stickstoffentwicklung wird dann das überschüssige Phosgen mit Stickstoff aus dem Reaktionsgemisch ausgeblasen und das Reaktionsgemisch wird durch Filtration von festen Bestandteilen befreit. Das Lösungsmittel wird unter vermindertem Druck und unter Ausschluß von Feuchtigkeit abdestilliert und der verbleibende Rückstand wird durch Destillation im Hochvakuum weiter gereinigt.

Die Erfindung wird durch die nachfolgenden Beispiele näher erläutert:

Beispiel 1:

27,3 mg (0,1 mMol) Natriumperrhenat und 1,04 g (5,25 mMol) Diphosgen wurden in 1 ml mit Chlorwasserstoff gesättigtem Essigsäureethylester bei 80 °C vorgelegt. Sodann wurden 1,71 g (10 mMol) 2-Azidohexansäuremethylester, gelöst in 5 ml Essigsäureethylester, unter kräftigem Rühren innerhalb 20 Minuten zugegeben. Die Umsetzung war nach 1 Stunde gleichmäßiger Gasentwicklung beendet. Das Reaktionsgemisch wurde dann über eine Vakuumfritte abgesaugt und der Essigsäureethylester unter vermindertem Druck abdestilliert. Das so erhaltene Rohprodukt wurde im Hochvakuum (0,0013 mbar) bei 50 bis 55 °C Gerätetemperatur im Kugelrohr destilliert. Es ergaben sich 1,35 g (79,8% der Theorie) analysenreiner 2-Isocyanato-2-hexensäuremethylester als farblose, leicht bewegliche Flüssigkeit.

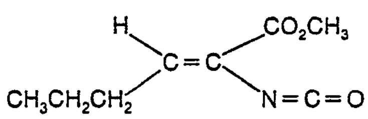

$C_8H_{11}NO_3$(169,18)

| | | | |
|---|---|---|---|
| Berechnet: | 56,80 %C | 6,55 %H | 8,28 %N |
| Gefunden: | 56,62 %C | 6,52 %H | 8,38 %N |

$^1$H-NMR ($CDCl_3$):

δ = 6,39 (t, 1H) $CH_2$–CH=;
3,85 (s, 3H) $OCH_3$;
2,28 (q, 2H) –$CH_2$–CH=;
1,81–1,14 (m, 2H) $CH_3$–$CH_2$–$CH_2$;
0,95 ppm (t, 3H) $CH_3$–$CH_2$.

IR $\nu_{NCO}$ = 2260 $cm^{-1}$,
$\nu_{CO}$ = 1725 $cm^{-1}$,
1655 $cm^{-1}$.

Beispiel 2:

27,3 mg (0,1 mMol) Natriumperrhenat und 1,04 g (5,25 mMol) Diphosgen wurden in 1 ml mit Chlorwasserstoff gesättigtem Essigsäureethylester bei 80 °C vorgelegt. Sodann wurden 2,05 g (10 mMol) 2-Azido-3-phenylpropansäuremethylester, gelöst in 5 ml Essigsäuremethylester, unter kräftigem Rühren innerhalb 20 Minuten zugegeben. Nach 1 Stunde Reaktionszeit bei 80 °C wurde analog Beispiel 1 aufgearbeitet. Das Rohprodukt wurde im Hochvakuum (0,0013 mbar) bei 90 bis 110 °C Gerätetemperatur im Kugelrohr destilliert. Es ergaben sich 1,08 g (53,2% der Theorie) analysenreiner 2-Isocyanato-3-phenylpropensäuremethylester.

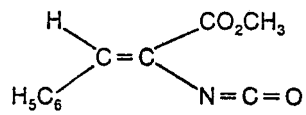

$C_{11}H_9NO_3$(203,197)

| | | | |
|---|---|---|---|
| Berechnet: | 65,02 %C | 4,46 %H | 6,89 %N |
| Gefunden: | 64,81 %C | 4,39 %H | 6,71 %N |

Schmelzpunkt: 39 bis 40 °C

$^1$H-NMR ($CDCl_3$):

δ = 7,95–7,22 (m, 5H) aromat. –CH=C;
7,10 (s, 1H) $C_6H_5$–CH=;
3,9 ppm (s, 3H) $OCH_3$.

IR $\nu_{NCO}$ = 2250 $cm^{-1}$,
$\nu_{C=O}$ = 1710 $cm^{-1}$,
1643 $cm^{-1}$.

Beispiel 3:

27,3 mg (0,1 mMol) Natriumperrhenat und 1,57 g (10 mMol) 2-Azido-3-methylbutansäuremethylester wurden in 5 ml Essigsäureethylester bei 80 °C in einem Frittenzylinder mit Gaseinleitungsrohr am Boden vorgelegt. Unter kräftigem Rühren wurde Phosgen durch die Reaktionslösung perlen gelassen. Nach 2 1/2 Stunden Reaktionszeit bei 80 °C wurde das überschüssige Phosgen mit Stickstoff aus der Lösung ausgeblasen und es wurde analog Beispiel 1 aufgearbeitet. Das Rohprodukt wurde im Hochvakuum (0,0013 mbar) bei 50 bis 55 °C Gerätetemperatur im Kugelrohr destilliert.

Es ergaben sich 1,37 g (88,2% der Theorie) analysenreiner 2-Isocyanato-3-methyl-2-butensäuremethylester als farblose, leicht bewegliche Flüssigkeit.

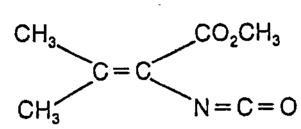

$C_7H_9NO_3$(155,153)

| | | | |
|---|---|---|---|
| Berechnet: | 54,19 %C | 5,85 %H | 9,03 %N |
| Gefunden: | 54,48 %C | 5,94 %H | 8,88 %N |

$^1$H-NMR ($CDCl_3$):

δ = 3,85 (s, 3H) $OCH_3$;

2,18 (s, 3H) 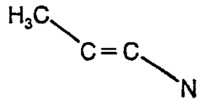 ;

2,01 ppm (s, 3H) 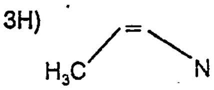 .

IR $\nu_{N=C=O}$ = 2250 $cm^{-1}$,
$\nu_{C=O}$ = 1713 $cm^{-1}$,
1630 cm.

Beispiel 4:

27,3 mg (0,1 mMol) Natriumperrhenat und 1,04 g (5,25 mMol) Diphosgen wurden in 5 ml mit Chlorwasserstoff gesättigtem Essigsäureethylester bei 80 °C vorgelegt. Sodann wurden 1,75 g (10 mMol) 2-Azido-3-methylmercapto-propansäuremethylester, gelöst in 20 ml Essigsäureethylester, unter kräftigem Rühren innerhalb 20 Minuten zugegeben. Nach 1 Stunde Reaktionszeit bei 80 °C wurde analog Beispiel 1 aufgearbeitet. Das Rohprodukt wurde im Hochvakuum (0,0013 mbar) bei 60 bis 70 °C Gerätetemperatur im Kugelrohr destilliert.

Es ergaben sich 1,43 g (82,6% der Theorie) analysenreiner 2-Isocyanato-3-methylmercapto-propensäuremethylester als farblose Flüssigkeit, die im Kühlschrank zu farblosen Kristallen erstarrte.

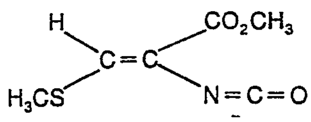

$C_6H_7NO_3S$(173,19)

| | | | |
|---|---|---|---|
| Berechnet: | 41,61 %C | 4,07 %H | 8,09 %N |
| | 18,51 %S | | |
| Gefunden: | 41,86 %C | 4,34 %H | 8,20 %N |
| | 18,38 %S | | |

$^1$H-NMR ($CDCl_3$):

δ = 7,07 (s, 1H) $CH_3S\text{-}CH=$;
3,85 (s, 3H) $OCH_3$;
2,46 ppm (s, 3H) $SCH_3$.

IR $\nu_{NCO}$ = 2255 $cm^{-1}$,
$\nu_{C=O}$ = 1710 $cm^{-1}$,
1600 $cm^{-1}$.

Beispiel 5:

72 mg (0,249 mMol) Kaliumperrhenat und 2,6 g (13,1 mMol) Diphosgen wurden in 5 ml Essigsäureethylester bei 80 °C vorgelegt. Sodann wurden 4,6 g (24,8 mMol) 2-Azidobutansäure-n-butylester, gelöst in 10 ml Essigsäureethylester, unter kräftigem Rühren innerhalb von 15 Minuten zugegeben. Die Umsetzung war nach 2$^1/_2$ Stunden gleichmäßiger Gasentwicklung beendet. Das Reaktionsgemisch wurde dann über eine Vakuumfritte abgesaugt und der Essigsäureethylester wurde unter vermindertem Druck abdestilliert. Das so erhaltene Rohprodukt wurde im Vakuum (0,13 mbar) bei 105 bis 115 °C Gerätetemperatur im Kugelrohr destilliert.

Es ergaben sich 2,7 g (59,4% der Theorie) analysenreiner 2-Isocyanato-2-butensäure-n-butylester.

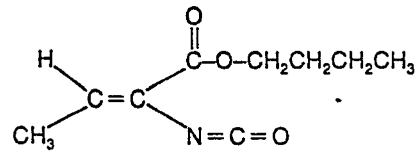

$C_9H_{13}NO_3$(183,21)

$^1$H-NMR ($CDCl_3$/TMS):

δ = 6,45 (q, 1H) $CH_3\text{-}CH=$;
4,2 (t, 1H) $O\text{-}CH_2\text{-}$;
1,85 (d, 3H) $CH_3\text{-}CH=$;
0,95 (t, 3H) $CH_2\text{-}CH_3$;
0,65–2,0 ppm (m, 4H) $-CH_2\text{-}CH_2\text{-}CH_3$.

IR $\nu_{NCO}$ = 2240 $cm^{-1}$,
$\nu_{CO}$ = 1715 $cm^{-1}$,
1660 $cm^{-1}$.

Beispiel 6:

68 mg (0,249 mMol) Natriumperrhenat und 2,5 g (12,6 mMol) Diphosgen wurden in 5 ml Essigsäureethylester bei 80 °C vorgelegt. Sodann wurden 4,6 g (24,8 mMol) 2-Azidopentansäure-isopropylester, gelöst in 10 ml Essigsäureethylester, unter kräftigem Rühren innerhalb von 30 Minuten zugegeben. Die Umsetzung war nach 2 Stunden gleichmäßiger Gasentwicklung beendet. Das Reaktionsgemisch wurde dann über eine Vakuumfritte abgesaugt und der Essigsäureethylester wurde unter vermindertem Druck abdestilliert. Das so erhaltene Rohprodukt wurde im Vakuum (0,067 mbar) bei 55 bis 60 °C Gerätetemperatur im Kugelrohr destilliert.

Es ergaben sich 2,95 g (64,9% der Theorie) analysenreiner 2-Isocyanato-2-pentensäure-isopropylester.

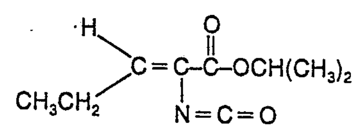

$C_9H_{13}NO_3$(183,21)

$^1$H-NMR ($CDCl_3$/TMS):

δ = 6,3 (t, 1H) $CH_2\text{-}CH=$;
4,7–5,4 (m, 1H) $O\text{-}CH\text{-}$;
2,85–2,65 (m, 2H) $-CH_2\text{-}CH$;
1,3 (d, 6H) $OCH(CH_3)_2$;
1,0 ppm (t, 3H) $CH_3\text{-}CH_2$.

IR $\nu_{NCO}$ = 2240 $cm^{-1}$,
$\nu_{CO}$ = 1715 $cm^{-1}$,
1650 $cm^{-1}$.

Beispiel 7:

72 mg (0,249 mMol) Kaliumperrhenat und 2,6 g (13,1 mMol) Diphosgen wurden in 3 ml mit Chlorwasserstoff gesättigtem Essigsäureethylester bei 80 °C vorgelegt. Sodann wurden 4,6 g (24,8 mMol) 2-Azido-hexansäureethylester, gelöst in 10 ml Essigsäureethylester, unter kräftigem Rühren innerhalb von 15 Minuten zugegeben. Die Umsetzung war nach 2 Stunden gleichmäßiger Gasentwicklung beendet. Das Reaktionsgemisch wurde dann über eine Vakuumfritte abgesaugt und der Essigsäureethylester wurde unter vermindertem Druck abdestilliert. Das so erhaltene Rohprodukt wurde im Vakuum (0,067 mbar) bei 90 bis 110 °C Gerätetemperatur im Kugelrohr destilliert.

Es ergaben sich 3,3 g (72,6% der Theorie) analysenreiner 2-Isocyanato-2-hexensäureethylester.

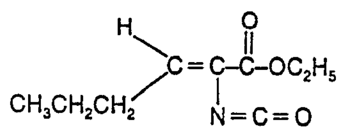

$C_9H_{13}O_3N$(183,21)

$^1$H-NMR ($CDCl_3$/TMS):

δ = 6,35 (t, 1H) $CH_2$–CH=;
4,25 (q, 2H) O–$CH_2$–;
2,2 (q, 2H) –$CH_2$–CH;
1,1–1,75 (m, 2H) $CH_3$–$CH_2$–;
1,25 (t, 3H) $OCH_2$–$CH_3$;
0,85 ppm (t, 3H) $CH_3$–$CH_2$.

IR $\nu_{NCO}$ = 2240 cm$^{-1}$,
$\nu_{CO}$ = 1715 cm$^{-1}$,
1650 cm$^{-1}$.

Beispiel 8:

72 mg (0,249 mMol) Kaliumperrhenat und 2,6 g (13,1 mMol) Diphosgen wurden in 5 ml Essigsäureethylester bei 80 °C vorgelegt. Sodann wurden 4,95 g (24,8 mMol) 2-Azido-octansäuremethylester, gelöst in 10 ml Essigsäureethylester, unter kräftigem Rühren innerhalb von 20 Minuten zugegeben. Die Umsetzung war nach 2,5 Stunden gleichmäßiger Gasentwicklung beendet. Das Reaktionsgemisch wurde dann über eine Vakuumfritte abgesaugt und der Essigsäureethylester wurde unter vermindertem Druck abdestilliert. Das so erhaltene Rohprodukt wurde im Vakuum (0,11 mbar) bei 105 bis 115 °C Gerätetemperatur im Kugelrohr destilliert.

Es ergaben sich 2,8 g (57,25% der Theorie) analysenreiner 2-Isocyanato-2-octensäuremethylester.

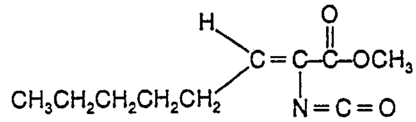

$C_{10}H_{15}NO_3$(197,2)

$^1$H-NMR ($CDCl_3$/TMS):

δ = 6,35 (t, 1H) $CH_2$–CH=;
4,85 (s, 3H) O–$CH_3$;
2,25 (dt, 2H) –$CH_2$–CH=;
0,65–1,80 ppm (m, 9H) $CH_3$–$CH_2$–$CH_2$–$CH_2$–.

IR $\nu_{NCO}$ = 2230 cm$^{-1}$,
$\nu_{CO}$ = 1725 cm$^{-1}$,
1650 cm$^{-1}$.

Beispiel 9:

68 mg (0,249 mMol) Natriumperrhenat und 2,6 g (13,1 mMol) Diphosgen wurden in 3 ml mit Chlorwasserstoff gesättigtem Essigsäureethylester bei 80 °C vorgelegt. Sodann wurden 5,6 g (24,6 mMol) 2-Azido-octansäureisopropylester, gelöst in 10 ml Essigsäureethylester, unter kräftigem Rühren innerhalb von 15 Minuten zugegeben. Die Umsetzung war nach 2 Stunden gleichmäßiger Gasentwicklung beendet. Das Reaktionsgemisch wurde dann über eine Vakuumfritte abgesaugt und der Essigsäureethylester wurde unter vermindertem Druck abdestilliert. Das so erhaltene Rohprodukt wurde im Vakuum (0,067 mbar) bei 130 bis 150 °C Gerätetemperatur im Kugelrohr destilliert.

Es ergaben sich 4,0 g (72,2% der Theorie) analysenreiner 2-Isocyanato-2-octensäureisopropylester.

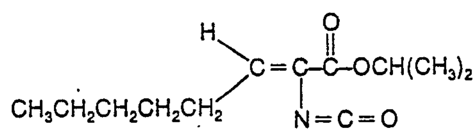

$C_{12}H_{19}NO_3$(225,3)

$^1$H-NMR ($CDCl_3$/TMS):

δ = 6,35 (t, 1H) $CH_2$–CH=;
4,75–5,45 (m, 1H) O–CH;
2,25 (dt, 2H) –$CH_2$–CH;
1,35 (d, 6H) $OCH(CH_3)_2$;
0,65–1,8 (m, 9H) $CH_3CH_2CH_2CH_2$–.

IR $\nu_{NCO}$ = 2240 cm$^{-1}$,
$\nu_{CO}$ = 1710 cm$^{-1}$,
1650 cm$^{-1}$.

Beispiel 10:

72 mg (0,249 mMol) Kaliumperrhenat und 2,6 g (13,1 mMol) Diphosgen wurden in 5 ml Essigsäureethylester bei 80 °C vorgelegt. Sodann wurden 5,25 g (24,85 mMol) 2-Azido-3-cyclohexyl-propionsäuremethylester, gelöst in 10 ml Essigsäureethylester, unter kräftigem Rühren innerhalb von 15 Minuten zugegeben. Die Umsetzung war nach 2,5 Stunden gleichmäßiger Gasentwicklung beendet. Das Reaktionsgemisch wurde dann über eine Vakuumfritte abgesaugt und der Essigsäureethylester wurde unter vermindertem Druck abdestilliert. Das so erhaltene Rohprodukt wurde im Vakuum (0,09 mbar) bei 140 bis 150 °C Gerätetemperatur im Kugelrohr destilliert.

Es ergaben sich 2,7 g (52,0% der Theorie) analysenreiner 2-Isocyanato-3-cyclohexyl-propensäuremethylester.

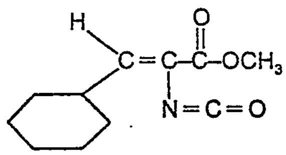

$C_{11}H_{15}NO_3$(209,25)

$^1$H-NMR ($CDCl_3$/TMS):

δ = 6,15 (d, 1H) CH=;
3,8 (s, 3H) O–$CH_3$;
0,45–2,85 ppm (m, 11H) 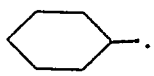.

IR $\nu_{NCO}$ = 2230 $cm^{-1}$,
$\nu_{CO}$ = 1720 $cm^{-1}$,
1645 $cm^{-1}$.

Beispiel 11:

27,3 mg (0,1 mMol) Natriumperrhenat und 2,0 g (10 mMol) Diphosgen wurden in 2 ml Essigsäureethylester bei 80 °C vorgelegt. Sodann wurden 1,8 g (9,13 mMol) 2-Azido-3-cyclopentylpropionsäuremethylester, gelöst in 2 ml Essigsäureethylester, unter kräftigem Rühren innerhalb von 20 Minuten zugegeben. Die Umsetzung war nach 3,5 Stunden gleichmäßiger Gasentwicklung beendet. Das Reaktionsgemisch wurde dann über eine Vakuumfritte abgesaugt und der Essigsäureethylester unter vermindertem Druck abdestilliert. Das so erhaltene Rohprodukt wurde im Vakuum (0,067 mbar) im Kugelrohr destilliert.

Es ergaben sich 1,3 g (66,6% der Theorie) 2-Isocyanato-3-cyclopentyl-propensäuremethylester.

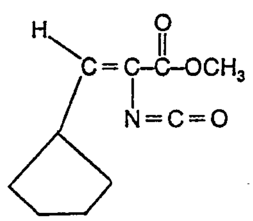

$C_{10}H_{13}O_3N$(195,21)

$^1$H-NMR ($CDCl_3$/TMS):

δ = 6,25 (d, 1H) CH=C;
3,80 (s, 3H) $COOCH_3$:
0,9–3,2 ppm (m, 9H) 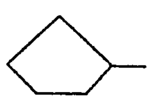

IR $\nu_{NCO}$ = 2230 $cm^{-1}$,
$\nu_{CO}$ = 1720 $cm^{-1}$,
1645 $cm^{-1}$.

Beispiel 12:

68 mg (0,249 mMol) Natriumperrhenat und 5,65 g (24,4 mMol) 2-Azido-3-cyclopentyl-propionsäure-isopropylester wurden in 12 ml Essigsäureethylester bei 80 °C vorgelegt. Unter kräftigem Rühren wurde Phosgen durch die Lösung geleitet. Nach 4 Stunden Reaktionszeit bei 80 °C wurde überschüssiges Phosgen mit Stickstoff aus der Lösung ausgeblasen. Das Reaktionsgemisch wurde dann über eine Vakuumfritte abgesaugt und der Essigsäureethylester unter vermindertem Druck abdestilliert. Das so erhaltene Rohprodukt wurde im Vakuum (0,13 mbar) bei 140 bis 150 °C Gerätetemperatur im Kugelrohr destilliert.

Es ergaben sich 3,5 g (64,2% der Theorie) 2-Isocyanato-3-cyclopentyl-propensäure-isopropylester.

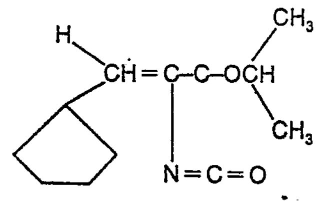

$C_{12}H_{17}O_3N$(223,27)

$^1$H-NMR ($CDCl_3$/TMS):

δ = 6,25 (d, 1H) CH=C;
5,75–5,45 (m, 1H) COOCH;
1,35 (d, 6H) $OCH(CH_3)_2$;
0,9=3,2 ppm (m, 9H) 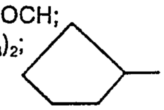

IR $\nu_{NCO}$ = 2230 $cm^{-1}$,
$\nu_{CO}$ = 1705 $cm^{-1}$,
1645 $cm^{-1}$.

Beispiel 13:

68 mg (0,249 mMol) Natriumperrhenat und 7,8 g (25,04 mMol) 2-Azido-hexadecansäuremethylester wurden in 12 ml Essigsäureethylester bei 80 °C vorgelegt. Unter kräftigem Rühren wurde Phosgen durch die Lösung geleitet. Nach 6 Stunden Reaktionszeit bei 80 °C wurde überschüssiges Phosgen mit Stickstoff aus der Lösung ausgeblasen. Das Reaktionsgemisch wurde dann über eine Vakuumfritte abgesaugt und der Essigsäureethylester unter vermindertem Druck abdestilliert. Das so erhaltene Rohprodukt wurde dann im Vakuum (0,067 mbar) im Kugelrohr destilliert.

Es ergaben sich 5,1 g (65,8% der Theorie) 2-Isocyanato-2-hexadecensäuremethylester.

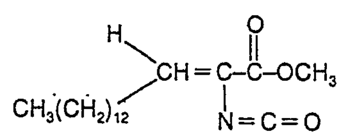

$C_{18}H_{31}O_3N$(309,45)

$^1$H-NMR ($CDCl_3$(TMS):

δ = 6,35 (t, 1H) CH=C;
3,85 (s, 3H) $COOCH_3$;
0,9–2,95 (m, 24H) $-(CH_2)_{12}-$;
0,85 ppm (t, 3H) $CH_3$–.

IR $\nu_{NCO}$ = 2230 $cm^{-1}$,
$\nu_{CO}$ = 17 $cm^{-1}$,
1645 $cm^{-1}$.

**Patentansprüche**

1. Verfahren zur Herstellung von 2-Isocyanato-2,3-dehydrocarbonsäureestern der allgemeinen Formel

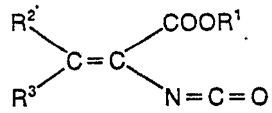

(I),

in der

$R^1$ einen geradkettigen oder verzweigten ($C_1$–$C_4$)-Alkylrest, einen Phenylrest oder einen Benzylrest,

$R^2$ Wasserstoff oder einen Methylrest und

$R^3$ Wasserstoff, einen geradkettigen oder verzweigten ($C_1$–$C_{16}$)-Alkylrest, einen ($C_3$–$C_8$)-Cycloalkylrest, einen ($C_1$–$C_6$)-Alkylmercaptorest oder einen Phenylrest

bedeuten, dadurch gekennzeichnet, daß man einen 2-Azidocarbonsäureester der allgemeinen Formel

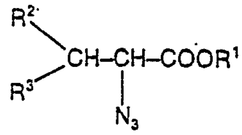

(II),

in der $R^1$, $R^2$ und $R^3$ die bereits angegebene Bedeutung haben, in einem inerten Lösungsmittel bei einer Temperatur zwischen 0 und 150 °C in Gegenwart eines Perrhenats mit Phosgen oder Diphosgen umsetzt.

2. Verfahren nach Anspruch 1, dadurch gekennzeichnet, daß man das Perrhenat in einer Menge zwischen 0,005 und 10 Molprozent, bezogen auf die Menge an eingesetztem 2-Azidocarbonsäureester der allgemeinen Formel (II), anwendet.

**Claims**

1. Process for the preparation of 2-isocyanato-2,3-dehydrocarboxylic acid esters of the general formula

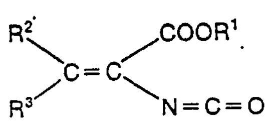

(I),

in which

$R^1$ denotes a straight-chain or branched ($C_1$–$C_4$)-alkyl radical, a phenyl radical or a benzyl radical,

$R^2$ denotes hydrogen or a methyl radical and

$R^3$ denotes hydrogen, a straight-chain or branched ($C_1$–$C_{16}$)-alkyl radical, a ($C_3$–$C_8$)-cycloalkyl radical, a ($C_1$–$C_6$)-alkylmercapto radical or a phenyl radical,

characterized in that a 2-azido-carboxylic acid ester of the general formula

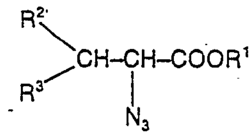

(II),

in which $R^1$, $R^2$ and $R^3$ have the meaning already given, is reacted with phosgene or diphosgene in an inert solvent at a temperature between 0 and 150 °C in the presence of a perrhenate.

2. Process according to claim 1, characterized in that the perrhenate is used in an amount of between 0.005 and 10 mol percent, based on the amount of 2-azidocarboxylic acid ester of the general formula (II) employed.

**Revendications**

1. Procédé de préparation d'esters 2-isocyanato-2,3-déhydrocarboxylates de formule générale

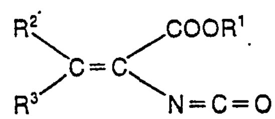

(I),

dans laquelle:

$R^1$ est un reste alkyle linéaire ou ramifié ($C_1$–$C_4$), un reste phényle ou un reste benzyle,

$R^2$ est l'hydrogène ou un reste méthyle et,

$R^3$ est l'hydrogène, un reste alkyle linéaire ou ramifié ($C_1$–$C_{16}$), un reste cycloalkyle ($C_3$–$C_8$), un reste alkylmercapto ($C_1$–$C_6$) ou un reste phényle, caractérisé en ce qu'on fait réagir un ester 2-azidocarboxylate de formule générale:

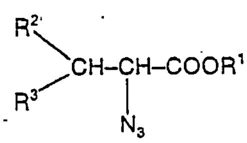

(II),

dans laquelle $R^1$, $R^2$ et $R^3$ ont la signification déjà indiquée, avec du phosgène ou du diphosgène en présence d'un perrhénate, dans un solvant inerte à une température comprise entre 0 et 150 °C.

2. Procédé selon la revendication 1, caractérisé en ce qu'on utilise le perrhénate en une quantité comprise entre 0,005 et 10 pourcents molaires, par rapport à la quantité du ester 2-azidocarboxylate de formule (II) utilisé.